# EUROPEAN PATENT APPLICATION

(11) **EP 4 516 270 A1**
(43) Date of publication of application: **05.03.2025**
(21) Application number: 23315337.8
(22) Date of filing: 04.09.2023
(51) Int. Cl.: A61F 5/00

(54) **DUODENAL ANCHOR FOR HOLDING IN PLACE AN IMPLANT IN A DUODENUM OF A PATIENT, AN IMPLANT FOR PLACEMENT IN A DUODENUM OF A PATIENT, A CONNECTOR FOR CONNECTING A DUODENAL ANCHOR AND A GASTRIC ANCHOR, AND AN IMPLANT SYSTEM FOR IMPLANTATION IN A PATIENTS GASTRIC TRACT, COMPRISING A GASTRIC ANCHOR, A DUODENAL ANCHOR, AND A CONNECTOR**

(71) Applicant: BariaTek Medical, 75013 Paris (FR)
(72) Inventor: GRAY, Yonatan, 75011 Paris (FR); NAZ, Christophe, 77300 Fontainebleau (FR); BIADILLAH, Youssef, 78600 Maisons-Laffitte (FR); BAERD, Leonard Raphaël, 75018 Paris (FR); POULETTY, Philippe, 75005 Paris (FR); PAU, Antoine, 75017 Paris (FR)
(74) Representative: Hepp Wenger Ryffel AG

(57) **Abstract**

The invention relates to an implant system (500) for implantation in a patient's gastric tract. The system comprises a gastric anchor (400) adapted to be placed in a patient's stomach, a duodenal anchor (100), a connector (300) having a first (360) and a second end (370) and a central portion (310). The connector (300) is attached to the duodenal anchor (100) at a first end (360), and connected to the gastric anchor at the second end (370). The first (360) and second ends (370) of the connector (300) are connected by a central portion (310) and adapted, when the system (500) is implanted, to connect the gastric anchor (400) and the duodenal anchor (100) across a patient's pylorus.

## Description

The invention is directed to a duodenal anchor for holding in place an implant in a duodenum of a patient, an implant for placement in a duodenum of a patient, a connector for connecting a duodenal anchor and a gastric anchor, an implant system for implantation in a patient's gastric tract, comprising a gastric anchor, a duodenal anchor, and a connector, and methods of treating a patient according to the preamble of the independent claims.

Implants for placement in a patient's gastrointestinal tract, such as duodenum and/or stomach, are known in the prior art.

EP 3 878 415 discloses a device for treatment of obesity or diabetes comprising a duodenal tube and a first anchor. The first anchor is adapted for anchoring the tube distally to the pylorus without mucosal involvement. A second anchor in the form of a conical, inflatable balloon is used for anchoring the device in the stomach of the patient.

EP 4 062 879 discloses an anchor for anchoring a tube with respect to a pylorus of a patient. The anchor may comprise a gastric bulb which is collapsible for introduction and optionally self-expanding to an operative condition.

US 2007/250132 discloses devices and methods for applying gastrointestinal stimulation which include implanting a stimulation device including a body with at least one expandable portion and a bridging portion and at least one stimulation member in the gastrointestinal tract.

WO 2011/099940 discloses a device that can be placed, retained, and/or anchored within a gastrointestinal tract. The device includes an anchor portion and a chute that is couplable to the anchor portion. The anchor portion includes a retention unit and can be shaped, dimensioned, and/or configured for retention in a trans-pyloric configuration.

The devices according to the prior art do all have certain disadvantages. In particular, they may be difficult to deploy and/or anchor, they may create an undesirable mucosal engagement, they may have unreliable anchoring, and they may have an unreliable transfer of chyme.

It is an object of the present invention to overcome the drawbacks of the prior art, in particular to provide implants and duodenal anchors for holding in place an implant in a duodenum of a patient which is easier to manufacture, easier to deploy, which offers a reliable anchoring and which reduces negative side effects such as unreliable transfer of chyme.

A further object of the invention is to provide implants and treatments for placement in a gastric tract of a patient which are easy to manufacture, easy to deploy, which offer a reliable anchoring and which does not have negative side effects, in particular due to unreliable transfer of chyme.

These and other objects are achieved with devices and methods according to the characterizing portion of the independent claims. Further embodiments result from the dependent patent claims.

According to the invention, a duodenal anchor for holding in place an implant in a duodenum of a patient is provided. The duodenal anchor has an outer surface which is configured to adapt to an inner wall of the patient's duodenum. The duodenal anchor comprises an inner, preferably central, passage for conveying chyme. The duodenal anchor further comprises a fillable part arranged at least partially circumferentially with respect to the inner passage. The fillable part, when fully filled with a fluid, has a larger circumference than an inner circumference of the duodenum.

The fillable part may be an inflatable part, in particular a balloon. An inflatable part may, in general, comprise a fluid-tight and/or gas-tight inner volume so that a fluid may be inserted, e.g. via a valve or a closable opening, and is prevented from exiting the volume.

Preferably, the fillable part may comprise or be formed by an outer jacket. The outer jacket may have a first end and a second end. The first end may be attached, in particular glued, to the inner passage such as to form an inner volume which may be fillable with a fluid. The outer jacket is attached with the first end, in a fluid-tight manner, to the inner passage.

The outer jacket may at least partially surround the inner passage.

The inner volume formed by the outer jacket and the inner passage does not need to be fluid-tight.

Preferably, the second end of the outer jacket is a free end adapted to move, e.g. slide, at least or preferably only along the surface of the inner passage. In other words, the outer jacket may be attached on one end, but not the other end, so that a fluid filled in the inner volume may escape the inner volume via a gap formed between the second end and the inner passage.

It will be understood that such an arrangement is still fillable with a fluid, even though the fluid is free the escape the inner volume.

Preferably, at least one connector passes through the fillable part and is attached to a distal end of the fillable part. Preferably, three connectors pass through the fillable part. Particularly preferably, the distal end to which the at least one connector is attached is the second end of the outer jacket. It will be understood that the connector described here may be formed by extensions of a connector which connects the duodenal anchor and a gastric anchor.

The at least one connector may be connected to the second end, pass through the inner volume of the fillable part, and pass through a hole in a proximal part of the outer jacket. The hole may be sealed in a fluid-tight manner and/or be adapted to that the connector may slide through the hole.

The at least one connector which passes through the fillable part may be an extremity of a connector as described herein below.

As a result, the second end may slide in the direction of the force leading to a volume reduction when a pulling force is exerted on the connector, e.g. due to peristaltic movements. The outer jacket may be folded, in an accordion-like manner. Fluid which was used to fill the fillable part may escape the inner volume due to reduction in volume by the folding.

While the filling with a fluid may be necessary for initial anchoring and provides advantageous adaptation to the inner shape of the patient's duodenum, it has been surprisingly found that the anchoring of the duodenal anchor is stronger when the outer jacket is folded up and the fluid is allowed to escape. Therefore, an automatic feedback mechanism may be provided which, when a force is exerted on the duodenal anchor, increased the anchoring by folding the outer jacket. As a result, accidental retrieval/motion of the duodenal anchor into the stomach may be avoided.

The duodenal anchor may also, preferably, comprise a pulling device. The pulling device may be attached to the distal end of the outer jacket. The pulling device may interact with the patient's intestine, e.g. due to peristaltic movements, so as to move away from the duodenal anchor and pull on the outer jacket.

In some patients, a gastric anchor attached to the duodenal anchor may exert a certain pulling force permanently. Therefore, when the duodenal anchor is configured to fold an outer surface (e.g. the outer jacket) due to said pulling force, there may be a bias that keeps the outer surface folded up permanently. While temporary folding is advantageous for the above-mentioned reasons, the folded configuration may irritate the duodenum over time. Therefore, it is desirable to revert the folding when no pulling force is present and/or when the pulling force is small enough that a folded structure is not necessary for sufficient anchoring. Furthermore, irritations may generally be reduced when the folded structure is allowed to move back and forth to some extent so as to adapt to the patient's duodenum. Therefore, the pulling device may reduce irritations by unfolding, at least partially, the outer jacket depending on the first pulling force acting on the pulling device and the second, opposite pulling force acting on e.g. the gastric anchor.

The invention is further directed to a pulling device for a duodenal anchor. The pulling device may be attached or attachable to a duodenal anchor, preferably directly, and preferably not be attached to a sleeve attached to the duodenal anchor.

Fully filled with fluid may be understood as being filled with a maximum quantity of fluid and/or gas without substantially increasing the pressure inside the fillable part above the pressure outside the fillable part (e.g. atmospheric pressure). Preferably, the fillable part can accommodate a maximum of 10 ml to 60 ml in volume of fluid (or gas) in its fully inflated and/or filled state. More preferably, the fillable part can accommodate a maximum of 25 ml to 45 ml in volume of fluid (or gas) when fully inflated and/or filled. In a preferred embodiment, the fillable part may hold a volume of 34 ml when fully inflated and/or filled.

In a particularly preferred embodiment, the fillable part is only filled with air (and/or another medically acceptable gas) for implantation and no additional fluid is inserted into the fillable part prior, during, or after implantation.

This allows the fillable part to reach the size of the duodenum without being fully inflated and/or filled. As a result, the fillable part may adapt to the inner surface of a duodenum, in particular its shape.

The fillable part may be filled by an incompressible fluid, such as a liquid (e.g. saline) and/or a gas (e.g. air). The use of air to fill the fillable part may result in a particularly soft structure when partially inflated.

A typical inner diameter of the human duodenum is 25 mm and a typical inner circumference of the duodenum 75 mm. The fillable part, when fully inflated and/or filled with a fluid and/or gas, may thus have a circumference larger than 75 mm and/or an inner diameter larger than 25 mm.

Preferably, the fillable part has an outer diameter in the range from 30 mm to 70 mm, more preferably in the range from 35 mm to 55 mm, most preferably in the range from 35 mm to 45 mm, when fully inflated and/or filled. In a preferred embodiment, the fillable part has an outer diameter, when fully inflated, of 40 mm.

Preferably, the fillable part is arranged circumferentially with respect to the inner passage which may be arranged centrally.

The outer surface of the duodenal anchor is configured to adapt to an inner wall of the patient's duodenum. To this end, the fillable part, in particular the outer wall, may comprise a material which is mechanically flexible, such as a soft polymer. Additionally or alternatively, the fillable part may be dimensioned such that at least an outer wall is mechanically flexible. The mechanical flexibility of the fillable part, in particular its outer wall, may be sufficient to adapt to the inner wall of the patient's duodenum. The fillable part and in particular the outer wall of the fillable part may be made of silicone, e.g. silicone with a Shore A hardness of 30-60. The tensile strain of the outer wall material of the fillable part and/or the tube/inner passage material may be 100-300%.

The inner passage may have a diameter in the range from 10 mm to 25 mm, preferably from 15 mm to 21 mm, most preferably from 17 mm to 19 mm. In a preferred embodiment, the inner passage has a diameter of 18 mm.

The duodenal anchor according to the invention is particularly suited to be delivered to an implant site and partially inflated at the implant site. Therefore, the anchor may have a compressed shape which is compact and allows for easy and safe delivery. Particularly preferably, the fillable part may be crimped under vacuum for delivery.

Partial inflation, i.e. the filling of the fillable part with an amount of fluid which does not lead to a fully filled state, may then be performed when the anchor is placed substantially at its final implant site. To this end, an inflation port may be present and in fluid communication with an inner volume of the fillable part. A static syringe may be used to inflate the fillable part. When the fillable part is vacuum crimped, the fluid may infiltrate the fillable part due to a pressure gradient and without additional pressure being provided on the syringe.

As the surrounding mucosa may constrain the fillable part before the fillable part reaches its nominal shape (i.e. before it is fully filled), the fillable part may reach an equilibrium which is under-inflated. At this point, the inflation port may be closed and an inflation tube may be disconnected from a valve.

As a result, the fillable part reaches a shape which corresponds to the anatomy at the implant site, i.e. the anchor can be adjusted to a patient specific shape on-site.

Such a patient specificity is achievable with an anchor which has a circumference which is larger than an inner circumference of a typical human duodenum because it allows for an under-inflated anchor to be securely implanted.

In general, the terms distal and proximal are used with respect to the direction of the gastrointestinal tract. A human stomach is at a proximal position with respect to the intestines. The intestines are at distal position with respect to a stomach. Accordingly, distal and proximal parts of any elements herein are meant to denominate their respective positions in an implanted position, i.e. oriented toward the stomach or intestines as described above.

In a preferred embodiment, the duodenal anchor has a cylindrical shape over at least part of its length. Preferably, the cylindrical shape extends over more than 40 % of its length. The length of the implant may be understood as an extension in a direction substantially parallel to a longitudinal axis of the duodenal anchor.
A cylindrical shape may in particular allow easy connection to other implants, for example a duodenal tube.

In a preferred embodiment, the duodenal anchor comprises a valve mechanism connected or connectable to the fillable part for filling the fillable part.

The valve mechanism may be connected to an inflation port. The valve mechanism may be adapted to receive an inflation tube which may open the valve mechanism when it is inserted. The valve mechanism may further be adapted to close when such a tube is removed.

In general, when the valve mechanism is in an open state, fluid may flow into the fillable part from an outside source which is connected or connectable to the inflation port and/or the valve mechanism. When the valve mechanism is closed, no fluid may exit the fillable part through the valve mechanism.

In a preferred embodiment, the duodenal anchor is attached to a proximal end of a sleeve, the sleeve having a distal end for placement within the small intestine.

In a preferred embodiment, the inner passage of the duodenal anchor is substantially tube shaped.

The inner passage of the duodenal anchor may be substantially tube shaped over at least 60 percent, more preferably 80 %, most preferably 90 % of its length along a direction substantially parallel to a longitudinal axis of the implant body.

In a preferred embodiment, the fillable part of the duodenal anchor comprises a mechanically flexible outer wall.

In a state where the duodenal anchor with the fillable part is placed in a duodenum of a patient, the fillable part is only partially filled. As a result of being only partially filled, the mechanically flexible outer wall of the fillable part may have an amorphic shape.

The mechanically flexible outer wall may provide several advantages. First, in a radial direction, it may succumb to the peristaltic motion of the bowels trying to push the outer wall of the fillable part back and forth. Second, in an axial direction, it may enable the mechanically flexible outer wall to have an amorphic shape, resulting from the partial inflation of the fillable part. As a result, only fluid within the fillable part may be moved back and forth, as a consequence of peristaltic motion of the bowels, instead of the whole duodenal anchor.

In a preferred embodiment, the duodenal anchor, preferably the fillable part, is crimped together by a release mechanism, preferably an crimping membrane and a release wire, to reduce a radial size of the duodenal anchor for delivery. Particularly preferably, the crimping membrane comprises or consists of silicone.

In order to facilitate delivery of the duodenal anchor inside the duodenum of a patient, it is advantageous to reduce the size, especially the radial size of the duodenal anchor.

The fillable part may be put under vacuum and crimped together by a release mechanism. The release mechanism may comprise a silicone crimping membrane tied together by a release wire adapted such that by pulling on an end of the release wire the crimping membrane releases the pressure exercised by it on the duodenal anchor.

In a preferred embodiment, the duodenal anchor comprises elongated elements with free distal ends that are extending in a direction substantially parallel to the longitudinal axis of the duodenal anchor. Such elongated elements may provide increased mechanical flexibility while preventing collapse, e.g. due to folding, of the sleeve surrounding it.

The distal end of the duodenal anchor may be facing the small intestine when implanted in a patient's duodenum.

The elongated elements may be made of a same or different material as the duodenal anchor. The elongated elements preferably comprise or consist of a soft material and may, additionally or alternatively, be dimensioned to be mechanically flexible (i.e. deform elastically under typical forces exerted in an implanted state). Elongated elements may provide stability to, e.g., a sleeve which is placed circumferentially around the elongated elements. It will be understood that the elongated elements may substantially have the same function as the elongated elements according to a preferred embodiment of the implant according to the invention. When the elongated elements are attached to the duodenal anchor, this may provide a particularly simple treatment because no separate implant needs to be provided.

Preferably, the elongated elements are arranged around the end of the inner passage at the distal end of the duodenal anchor. When extending along a direction parallel to a longitudinal axis of the duodenal anchor, the elongated elements preferably form a structure having the shape of a tube with straight longitudinal cuts. Preferably, each cut has the same width as each of the elongated elements.

In a preferred embodiment, the duodenal anchor is connectable, at a proximal end, to one of a connector and a stent, preferably by a plurality of connection elements arranged at a circumferential position with respect to the central passage.

Preferably, the duodenal anchor comprises at least one channel extending through a wall which is adapted for receiving a connector.

The proximal end of the duodenal anchor is facing the stomach when implanted in a patient's duodenum. The distal end of the duodenal anchor is opposite the proximal end.

Where a gastric anchor formed as a balloon is used to treat a patient, the duodenal anchor may also be connected or connectable to a gastric balloon.

According to another aspect of the invention, an implant for placement in a duodenum of a patient is provided. The implant having a generally tube-shaped implant body with a proximal end and a distal end. The proximal end of the implant body is fixable or fixed to a duodenal anchor, preferably a duodenal anchor as previously described. The implant body comprises a plurality of elongated elements with free distal ends extending in a direction substantially parallel to a longitudinal axis of the implant body. The free ends of the elongated elements form a distal-most portion of the implant body.

Preferably, the proximal end of the implant body is fixable or fixed to a duodenal anchor, more preferably to the distal end of the duodenal anchor. The distal end of the duodenal anchor may be facing the small intestine when implanted in a duodenum of a patient.

The implant body may be generally tube-shaped over at least 60 percent, more preferably 80 %, most preferably 90 % of its length along a direction substantially parallel to a longitudinal axis of the implant body.

The generally tube-shaped implant body together with the elongated elements may have a castle-like shape.

Circumferential spaces between two adjacent elongated elements may form a passage for chyme as the elongated elements will bend and kink with the anatomy of the patient. The circumferential spaces between the elongated elements may allow reliable transfer of chyme even when the elongated elements are bent and/or kinked, for example due to the anatomy of the patient. As a result, an chyme channel formed by the tube-like structure is less prone to blocking due to e.g. kinking.

Preferably, the width of each circumferential space between the longitudinal elements equals the width of each one of the elongated elements.

The circumferential spaces may be gaps.

The length of each elongated element may be between 5 mm and 30 mm, preferably between 10 mm and 15 mm.

Preferably, each elongated element has the shape of a rectangular cuboid. Preferably, the width of the elongated elements is larger than the thickness of the elongated elements. Preferably, the length of the elongated elements is larger than both, the width and the thickness of the elongated elements.

Preferably, the elongated elements comprise or consist of the same material as the implant body. Additionally or alternatively, the elongated elements comprise or consist of a mechanically softer material than the implant body.

Preferably, the elongated elements comprise or consist of durable thermoplastic such as thermoplastic polyurethane (TPU) and/or silicone. The material may have a Shore A hardness of 60.

Preferably, the elongated elements are arranged around a circle with the same diameter as the inner passage forming an extension of the tube-like shape.

Each of the elongated elements may have a width between 2 and 15 mm, preferably between 4 and 8 mm, particularly preferably between 5 and 6 mm.

In an advantageous embodiment, the implant further comprises a, preferably intestinal, sleeve arranged around the tube-shaped implant body and attached to the proximal end of the implant body, wherein a distal end of the sleeve is configured for placement within the small intestine. The sleeve may be adapted for transport of chyme and be adapted to reduce or prevent contact of chyme with an intestine wall.

The sleeve may be placed at a distance of 60 to 100 cm of the proximal bowels, i.e. the sleeve may cover the duodenum and parts of the jejunum. The diameter of the sleeve may be between 15 and 30 mm, preferably between 20 and 25 mm.

In an advantageous embodiment, the implant body comprises 3 to 20, preferably 6 to 12, elongated elements.

In an advantageous embodiment, the implant comprises or consists of a soft material, preferably an elastic soft material.

Preferably the elongated elements and the implant body comprise or consist of the same material.

Particularly preferably, the elongated elements consist of a soft material, preferably an elastic soft material, such as medical grade silicone.

The material may be a material with a Shore A hardness of less than 55-65.

In an advantageous embodiment, the implant has an outer dimension, in a direction perpendicular to a longitudinal axis, between 1 and 10 cm, preferably between 10 and 30 mm.

In an advantageous embodiment, the elongated elements have a closed surface, in particular in a direction perpendicular to a longitudinal axis.

A closed surface may be understood as being solid elements in the sense that they are filled with material, i.e., for example, they do not have holes through them.

For example, an elongated element having the shape of a rectangular cuboid, where the elongated element is filled with material (i.e. no holes), exhibits four closed surfaces in a direction perpendicular to a longitudinal axis.

Preferably, the elongated elements of the implant exhibit a closed surface in a direction perpendicular to a longitudinal axis, whereas the closed surface is the largest surface of the elongated element.

In an advantageous embodiment, at least one circumferential space, preferably all circumferential spaces, between the elongated elements of the implant body have a width corresponding to the width of the elongated elements. Where not all elongated elements have the same width, the width of the space may correspond to the width of at least one elongated element.

In an advantageous embodiment, the elongated elements of the implant body are impermeable to liquids and gases.

Impermeable to liquids and gases means that it does not allow the passage of liquids and gases or at least reduces the rate at which liquids or gases can pass through.

In an advantageous embodiment, the generally tube-shaped implant body has walls without openings and/or has a substantially contiguous surface.

The elongate elements may in particular have a straight shape without curves along the longitudinal axis. The elongate elements may consist of a soft material and/or of the same material as the implant body.

A solid rectangular cuboid may form a wall without openings.

According to still another aspect of the invention, a connector for connecting a duodenal anchor and a gastric anchor is provided. Preferably, the duodenal anchor is a duodenal anchor as previously described. The connector has a central portion and a first and second plurality of extremities arranged at opposite ends of the central portion. The central portion has a generally elongated shape along a longitudinal axis. The first plurality of extremities is configured to be fixed or fixable to the duodenal anchor at first extremity free ends. The second plurality of extremities is configured to be fixed or fixable to the gastric anchor at second extremity free ends. The first plurality and second plurality free ends extend away, in a radial direction, from the longitudinal axis. Preferably, the first plurality and second plurality free ends have connecting means for connection to a duodenal anchor and a gastric anchor, respectively.

The connector according to the invention allows to connect two elements, such as the gastric anchor and the duodenal anchor, through a pylorus while still allowing chyme to pass therethrough.

The connector may comprise or consist of medical-grade silicone which provides biocompatibility and is atraumatic.

The gastric anchor may be a stent or an inflatable gastric anchor such as a balloon. The inflatable gastric anchor may be fully inflated.

Where the gastric anchor is inflatable, the inflatable part may have a nominal volume of 30 to 80 ml, preferably 50 to 60 ml.

Where the gastric anchor is a stent, a stent length may be in the range of 15 to 50 mm, preferably 20-30mm. A diameter of the stent may be in the range of 30 to 80 mm, preferably 50 to 60 mm. The stent may comprise 9-18 stent cells, preferably 12-15.

Wires of the stent may extend in the pylorus with a length of 5 to 50 mm, preferably 20 to 30 mm. The number of wires may be between 2 and 6, preferably between 3 and 4.

The tensile elasticity of the wires may be up to 300%, whereas the assembled wires (which typically may include three wires which may also be glued or tied to duodenal and/or gastric anchors), the tensile elasticity is preferably in the range of 20 to 100%, particularly preferably 60 to 80%.

The gastric anchor may have a length to diameter ratio of 1:5 - 1:1, preferably 1:2 to 1:1, particularly preferably 8:11. In some embodiments, the length to diameter ratio may also be 1:2.

The duodenal anchor may be an inflatable or a non-inflatable duodenal anchor. The duodenal anchor may also comprise inflatable parts and non-inflatable parts.

Preferably, the central portion has a generally elongated shape and extends in a parallel direction along the longitudinal axis of the connector.

Preferably, the first plurality of extremities is configured to extend inside the duodenal anchor, whereas it may be casted in the duodenal anchor.

Preferably, the second plurality of extremities is configured to extend inside the gastric anchor and may be cast in the gastric anchor.

In a particularly preferred embodiment, at least one of the first and second plurality of extremities of the connector is formed by rods, preferably exactly three rods.

Preferably, the rods of the first plurality of extremities extend through the central portion and are connected to, or form, the second plurality of extremities.

Preferably, the first and/or second plurality of extremities is formed by 2 to 6 rods, more preferably by 3 to 4 rods.

Preferably, the rods of the first and second plurality of extremities have a tensile elasticity of 20% to 100%, more preferably of 60% to 80%.

In a particularly preferred embodiment, at least one of the first plurality free ends and the second plurality free ends of the connector are arranged at substantially constant angles and/or radial distances with respect to the longitudinal axis.

The first and/or second plurality free ends may all end on a virtual circle that is centered and normal to the longitudinal axis.

In a particularly preferred embodiment, the central portion of the connector, in a direction along the longitudinal axis, extends over a length between 3 and 50 mm, preferably between 20 mm and 30 mm. Generally, the central portion may have a length which is at least the length of a typical pyloric sphincter and may preferably have a length which is shorter than twice the typical pyloric valve.

In a particularly preferred embodiment, the lengths of the first plurality of extremities and/or the second plurality of extremities of the connector is between 10 and 50 mm, preferably between 20 and 30 mm.

These lengths allow the connector to partially extend beyond the pylorus valve and thus reduces or avoids abrasion of anchor shafts against the pylorus.

In some embodiments, the distance between the first and second extremities in a direction along a longitudinal axis is 20 to 30 mm.

In a particularly preferred embodiment, the first plurality of extremities and/or the second plurality of extremities and/or the central portion of the connector comprise or consist of a soft material.

A soft material is a material having a low hardness and a high flexibility. For example, silicone and/or polyurethane may be suitable materials. Preferably, the soft material has a tensile elasticity of 20% to 100%, more preferably of 60% to 80%. Preferably, the soft material has a Shore A hardness between 40 and 70, more preferably between 55 and 65.

Particularly preferably, the first plurality of extremities and/or the second plurality of extremities and/or the central portion (which may represent a transpyloric soft section) of the connector comprise or consist of elastic wires which may be elastically stretched up to 500-600% tensile strain at break. For cyclic loading on such an assembly (cyclic fatigue of the elastic wires), up to 150-200% strain without failure is conceivable.

In a particularly preferred embodiment, the central portion of the connector is formed by a plurality of central extensions and a circumferentially arranged tubular element. The central portion may be formed by a plurality of central extensions and a tubular element (350) which is arranged circumferentially around the central extensions. The tubular element may have a length at least as long as its diameter. The tubular element may be configured to reduce the lateral diameter of the central portion and/or such as to bring the central extensions into physical contact with each other.

The length of the tubular element may be measured along a direction parallel to a longitudinal axis of the tubular element and may have the same length as the central portion. Preferably, the tubular element has a length between 3 and 50 mm, more preferably between 5 and 30 mm, particularly preferably 5 to 15 mm, even more preferably 6 to 8 mm.

Preferably, the tubular element has a wall thickness between 0.2 and 4 mm, more preferably between 0.5 and 2 mm.

Preferably, the tubular element comprises or consists of the same material as the first and/or second plurality of extremities and/or the central portion of the connector. The tubular element may comprise or consist of medical silicone.

The plurality of central extensions may be 2 to 6 rods connected or connectable to the first and second plurality of extremities.

The plurality of central extensions may comprise a smaller number of central extensions than the first and/or second plurality of extremities comprises extremities.

Each central extension of the plurality of central extensions may be connected or connectable to more than one extremity of the first plurality of extremities.

Each central extension of the plurality of central extensions may be connected or connectable to more than one extremity of the second plurality of extremities.

Preferably, the tubular element is configured to surround all elements of the central portion. More preferably, the tubular element is configured to surround the plurality of central extensions.

According to still another aspect of the invention, an implant system for implantation in a patient's gastric tract is provided. The implant system comprises a gastric anchor, preferably one of a stent and a balloon, adapted to be placed in a patient's stomach. The implant system further comprises a duodenal anchor, preferably a duodenal anchor as previously described. The implant system further comprises a connector, preferably a connector as previously described. The connector has a first and a second end and a central portion. The connector is connected or connectable to the duodenal anchor at a first end, and connected or connectable to the gastric anchor at the second end. The first and second ends of the connector are connected by a central portion. The connector is adapted, when the system is implanted, to connect the gastric anchor and the duodenal anchor across a patient's pylorus.

It will be understood that in some embodiments it is conceivable to use a non-inflatable duodenal anchor.

Preferably, the connector is adapted to connect the gastric anchor and the duodenal anchor. The connector may already connect the gastric anchor and the duodenal anchor before the system is implanted, or they may be connected during a surgical treatment, e.g. when in a patient.

The length of the duodenal anchor may be 20 to 80 mm, preferably 30 to 50 mm or 50 to 60 mm, in along a direction in which chyme goes through the duodenal anchor/system. The length may include elongated elements as part of the duodenal anchor and/or an implant body with elongated elements which is connected to the duodenal anchor.

The gastric anchor, whether formed as a stent or a balloon, may have length in the range of 15 to 50 mm, preferably 30-50 mm or 20-30 mm. A diameter of the gastric anchor may be in the range of 30 to 80 mm, preferably 50 to 60 mm. Where the gastric anchor is formed by a balloon, the nominal volume of the gastric anchor may be 40 ml in some embodiments, or generally anywhere in the range of 50 to 80 mL, particularly preferably 70 mL.

The length of the connector, which is adapted to extend across the pylorus, may be between 5 and 50 mm, preferably 20 to 30 mm.

In a preferred embodiment, the implant system further comprises an implant, preferably an implant as previously described. The implant comprises an implant body. The implant body may be tube shaped. The implant body may be attached to the duodenal anchor at a proximal end. Preferably, the implant body comprises elongated elements extending in a direction away from the duodenal anchor.

In a preferred embodiment, the implant system further comprises a sleeve arranged around the tube-shaped implant body. The sleeve is attached to the proximal end of the implant body, whereas a distal end of the sleeve is configured for placement within the small intestine.

According to still another aspect of the invention, a method of treating a patient comprising the implantation of an implant system as previously disclosed is provided.

The method of treating a patient may comprise any one of the following steps:
- deposition of a guide wire in the proximal duodenum via an endoscope work channel;
- insertion of a concentric delivery system loaded with the implant system as previously disclosed having the distal tip of the delivery system ending with an atraumatic ball, over the deposited guide wire;
- pushing the delivery system through the patient throat and esophagus until reaching the stomach with the portion having the loaded docking station such that the ball and some of the sleeve may be distal to the pylorus;
- inserting an endoscope into the stomach for imaging purposes;
- under visual guidance of the endoscope, advancing the docking station forward, having the duodenal member passing the pylorus;
- pushing the sleeve distally via an internal catheter at the rear end of the delivery system, until sleeve is fully deployed in the small intestines;
- release of the distal atraumatic ball;
- injecting pressurized fluid through the sleeve via the internal catheter while retrieving the catheter. Sleeve is to reach full patency, which may be confirmed via X-ray imaging;
- release of a duodenal anchor crimp sleeve and partial inflation to desired volume
- un-sheath a gastric stent in the antrum (distal stomach) if the system comprises a gastric anchor formed by a stent or inflate the gastric anchor to its nominal volume if the gastric anchor is formed by a balloon;
- disconnection of balloons once anchor position and deployment is visually confirmed via the endoscope;
- retrieval of the endoscope;
- retrieval of the delivery system.

According to still another aspect of the invention, a method of treating a patient is disclosed. The method of treating a patient comprises the step of placing a duodenal anchor comprising a fillable part, preferably a duodenal anchor as previously described, in a patient's duodenum. The method of treating a patient further comprises a step of filling a predetermined quantity of fluid into the fillable part. The predetermined quantity of fluid is between 10% and 80% of a volume of the fillable part when fully inflated. The fillable part may accommodate, when fully inflated, a volume of 10 ml to 60 ml, preferably, 25 ml to 45 ml, particularly preferably 34 ml.

In a preferred embodiment, the predetermined quantity of fluid of the method of treating a patient is between 5 and 10 ml. However, it will be understood that any amount of liquid smaller than 50 ml, preferably less than 20 ml, particularly preferably less than 10 ml, may be suitable, depending on the size of the duodenal anchor that is used, and may lead to only partial inflation. In some embodiments, 1 to 5 ml are used.

The duodenal anchor may be retrievable from a patient's duodenum after implantation. Retrievability of the duodenal anchor allows to remove the duodenal anchor from a patient's duodenum, for example after a successful treatment of the patient.

The duodenal and/or gastric anchor may be adapted to filled, unfilled, inflated or deflated after implantation. To this end, the invention is further directed to a method of treating a patient, comprising the steps of placing a duodenal anchor, e.g. a duodenal anchor as described herein, and/or an inflatable gastric anchor, in the patients duodenum and/or gastric tract, respectively, wherein at least one of the duodenal anchor and the gastric anchor comprises an inflatable part. The method further comprises the step of inflating or deflating the inflatable part.

The implant system according to the invention and/or any element of the implant system (e.g. gastric anchor, duodenal anchor, sleeve, connector) may be placed by an autonomous robot. To this end, the invention is further directed to a method of treating a patient, comprising the steps of placing an implant system, e.g. an implant system as described herein, preferably a duodenal anchor, e.g. a duodenal anchor as described herein, and/or a gastric anchor, in the patients duodenum and/or gastric tract, respectively. The method comprises the step of positioning the duodenal anchor and/or the gastric anchor inside a patient's duodenum by a robot, preferably the robot is at least partially autonomous. The robot may be adapted to inflate and/or deflate the gastric anchor and/or the duodenal anchor. The robot may also place a sleeve which is attached to the duodenal anchor.

The implant system may comprise at least one sensor, in particular a biosensor, which is adapted to collect information, e.g. relating to a content of nutrients (like glucose and/or lipids) in chyme passing through the implant system. Additionally or alternatively, the sensor may be adapted to sense a shape and/or size of any part of the implant system, e.g. the duodenal anchor or gastric anchor, e.g. to determine whether the implant system is placed correctly at any point after implantation. Such information may be used within the duodenal anchor in a closed loop feedback and/or may be transmitted externally, e.g. via wireless communications for subsequent use by a care person. In particular, it may be possible to selectively change the size and shape of the duodenal anchor in response to the amount of nutrients measured in the stomach and/or in the intestine.

The invention is now described with reference to embodiments and figures which show:
- Figure 1:: schematic representation of a first embodiment of a duodenal anchor.
- Figure 2:: schematic representation of a second embodiment of a duodenal anchor.
- Figure 3:: schematic representation of an embodiment of the first embodiment of a duodenal anchor connected to an implant.
- Figure 4:: schematic representation of an embodiment of an implant.
- Figure 5:: schematic representation of an embodiment of a connector.
- Figure 6:: schematic representation of an embodiment of the connector connected to a duodenal anchor.
- Figure 7:: schematic representation of an embodiment of an implant system.
- Figure 8:: representation of another embodiment of the implant system.
- Figure 9:: schematic representation of another embodiment of the implant system.
- Figure 10:: representation of an embodiment of a duodenal anchor crimped by a release mechanism.
- Figure 11:: representation of an embodiment of a duodenal anchor in a deflated state.
- Figure 12a-12c:: a third embodiment of an implant with a duodenal anchor in different perspectives.
- Figure 13:: a schematic cross-sectional view of the duodenal anchor of Fig. 12a.
- Figure 14a-14b:: schematic representation of a working mechanism of the duodenal anchor of Fig. 13.
- Figure 15:: perspective view of a implant system having a pull device.
- Figure 16a-16b:: schematic representation of a working mechanism of the pull device of Fig. 15.

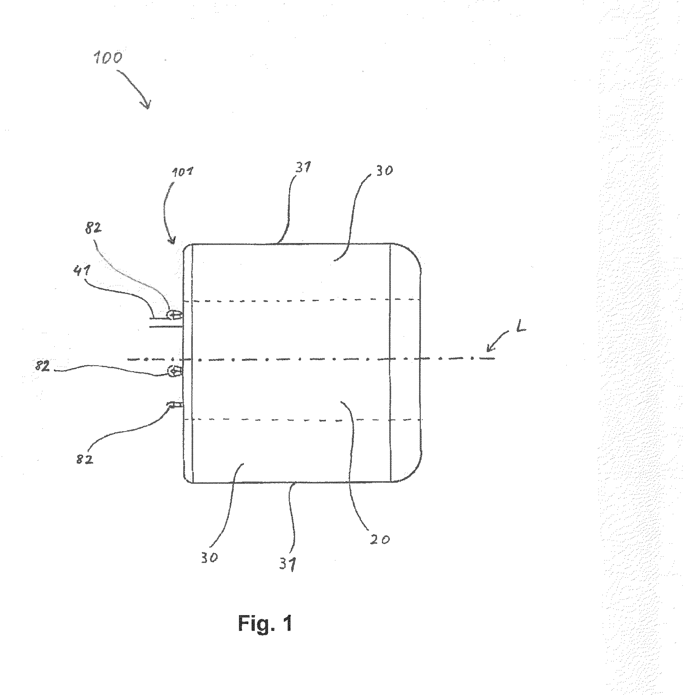

Figure 1 shows a first embodiment of a duodenal anchor 100. The duodenal anchor 100 comprises an inner passage 20 for conveying chyme, which is illustrated by two dotted lines. The inner passage 20 has the shape of a tube and is centered around a longitudinal axis L of the duodenal anchor 100. An inflatable part 30 is arranged around the inner passage 20. The inflatable part 30 has a mechanically flexible outer wall 31 that is configured to adapt to an inner wall of the patient's duodenum. The inflatable part 30 is connected to a valve mechanism 41. The duodenal anchor 100 has a proximal end 101 with connection elements 82 which are adapted to connect to e.g. a connector and/or a gastric anchor (not shown). The duodenal anchor shown here consists of silicone.

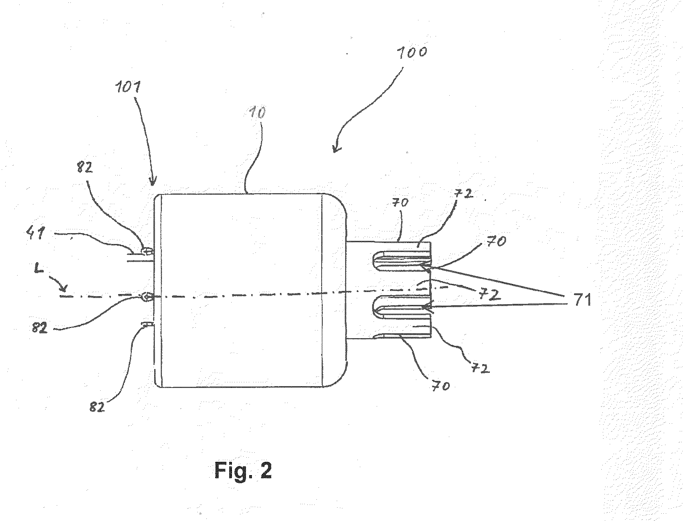

Figure 2 shows a further embodiment of a duodenal anchor 100. The duodenal anchor 100 is similar to the embodiment shown in Fig. 1. For clarity, identical features are not described separately here. Here, the duodenal anchor 100 is made of polyurethane and further comprises elongated elements 70 with free ends 72 and separated by gaps 71 between the free ends. Alternatively, the duodenal anchor 100 may be made of silicone. The duodenal anchor 100 has a mechanically flexible outer wall 10 that is configured to adapt to an inner wall of the patient's duodenum.

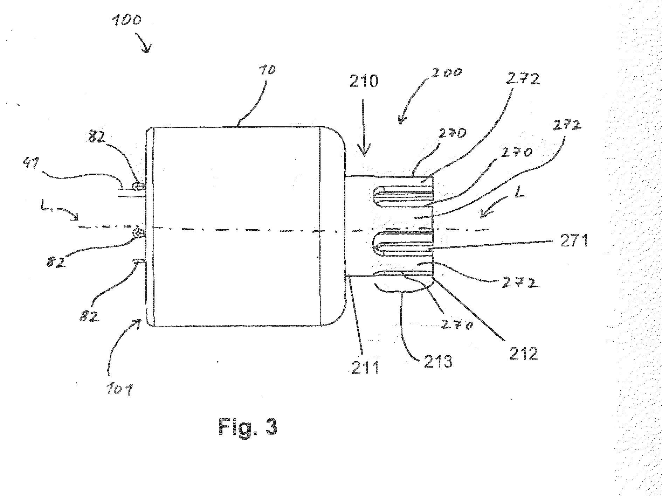

Figure 3 shows a schematic representation of an implant 200 connected to a duodenal anchor 100 as shown in Fig. 1. The implant 200 has a tube-shaped implant body 210 with a proximal end 211 and a distal end 212. The proximal end 211 is attached to the duodenal anchor 100. A plurality of elongated elements 270 with free distal ends 272 extend in a direction substantially parallel to the longitudinal axis L. The plurality of elongated elements 270 form a distal-most portion 213 of the implant body 210. Between the elongated elements 270, in a direction following the circumference of the implant body 210, there is a circumferential space 271. The duodenal anchor 100 has a mechanically flexible outer wall 10 that is configured to adapt to an inner wall of the patient's duodenum.

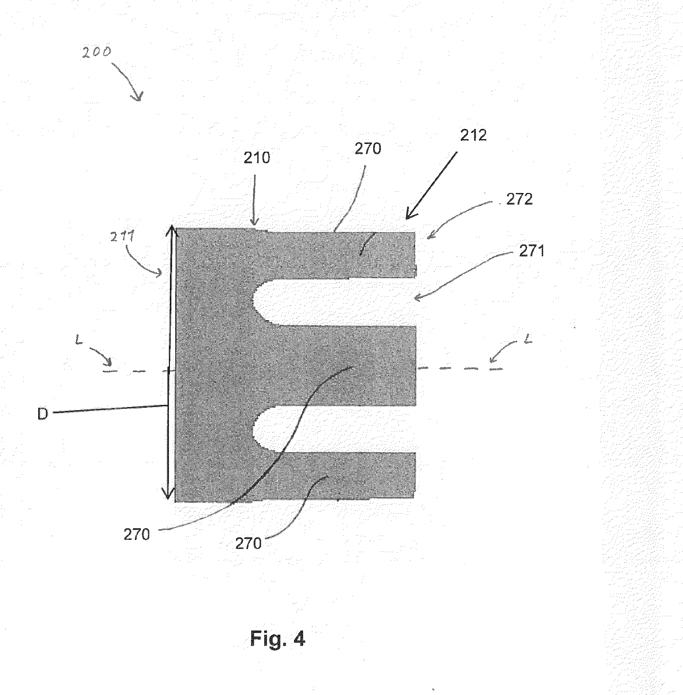

Figure 4 schematically shows an implant 200 according to the invention substantially formed by an implant body 210. At a proximal end 211, the implant body is formed substantially as a tube with closed lateral surface. From the proximal end 211 in a generally distal direction along the longitudinal axis L (from left to right as shown in Fig. 4), elongated elements 270 with free distal ends 272 are formed and separated by gaps 271 therebetween. The elongated elements 270 substantially form the distal end 212 of the implant body 210. The implant is made of medical-grade silicone. The implant body 210 shown here has an outer dimension D, in a direction perpendicular to the longitudinal axis L, of 5 cm. Here, the elongated elements 270 have closed surfaces and do not have any holes or discontinuities. The elongated elements are substantially solid and integrally formed extensions.

It will be understood that the implant of Fig. 4 combined with the duodenal anchor of Fig. 1 may result in a device which is functionally similar to the embodiment of Fig. 2 which is integrally formed.

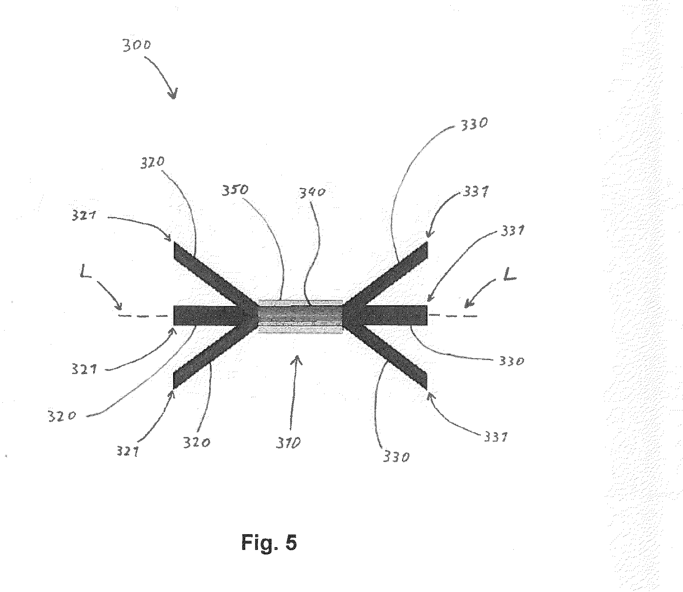

Fig. 5 shows a schematic representation of an embodiment of a connector 300. The connector 300 has a central part 310, a first plurality of extremities 320 and a second plurality of extremities 330. Preferably, the first plurality of extremities 320 and the second plurality of extremities 330 are formed by rods. The first and second plurality of extremities 320, 330 are terminated by first and second free ends 321, 331, respectively. The first plurality of extremities 320 and the second plurality of extremities 330 are connected by a plurality of central extensions 340, which are formed integrally in the present example, that extend throughout the central part 310. A tubular element 350 is circumferentially arranged around the central extensions 340 in the central part 310. The tubular element 350 extends over the whole length of the central part 310 and its length is considered in a direction along the longitudinal axis L of the connector 300. The plurality of central extensions 340 may, additionally or alternatively, be glued together in the central part.

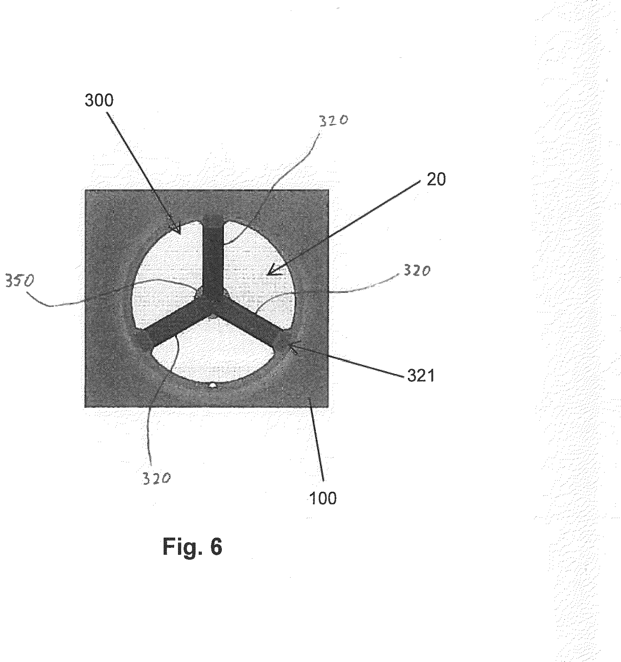

Figure 6 shows a schematic representation of an embodiment of the connector 300 connected to the duodenal anchor 100. Shown is a view along the longitudinal axis of the connector (see Fig. 5). The first plurality of extremities 320 of the connector 300 extend from the central part (not shown) surrounded by the tubular element 350. The first extremity free ends 321 are connected to the duodenal anchor 100 at a circumferential positions of the inner passage 20 at the proximal end of the duodenal anchor 100. The first plurality of extremities 320 are arranged at substantially constant angles and radial distances with respect to the longitudinal axis of the connector.

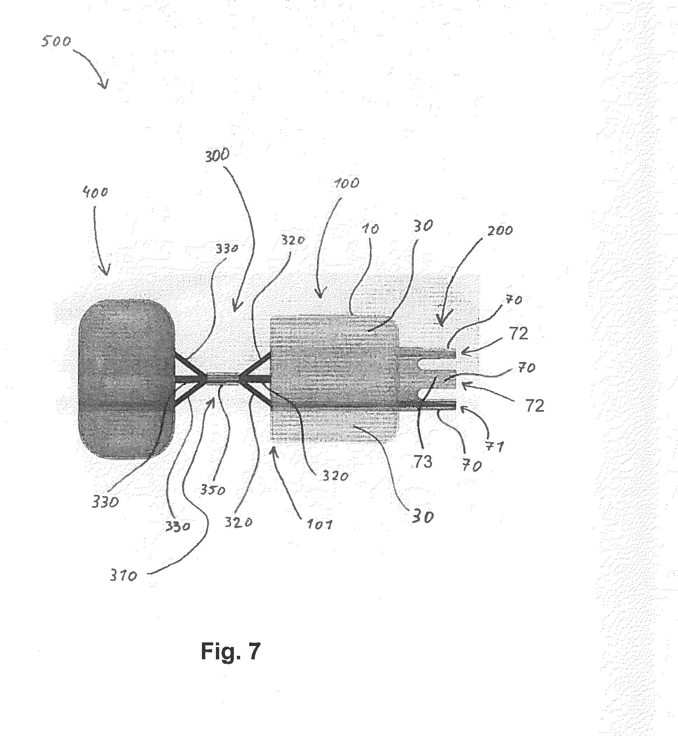

Figure 7 shows a schematic representation of an embodiment of an implant system 500. The implant system 500 comprises a gastric anchor 400, a duodenal anchor 100 having an inflatable part 30, similar to the one shown in Fig. 1, a connector 300 similar to the one shown in Fig. 5, and an implant 200 similar to the implant shown in Fig. 4. The implant 200 comprises elongated elements 70 with free distal ends 72. The elongated elements 70 have closed surfaces 73, i.e. the elongated elements 70 are filled with material. The implant 200 is connected to the distal end of the duodenal anchor 100. The distal end of the duodenal anchor is on the opposite side of the proximal end 101 of the duodenal anchor 100. The duodenal anchor 100 has an outer surface 10 which is configured to adapt to an inner wall of the patient's duodenum. At the proximal end 101 of the duodenal anchor 100, the first plurality of extremities 320 of the connector 300 are connected to the duodenal anchor 100. From the proximal end 101 of the duodenal anchor 100 towards the central part 310, the first plurality of extremities 320 are pulled together due to the tubular element 350. Portion covered by tubular element 350 has a length of 7 mm. The second plurality of extremities 330 of the connector 300 are connected to the gastric anchor 400. The gastric anchor 400 shown here is an inflatable balloon, but may be replaced by a stent in some embodiments.

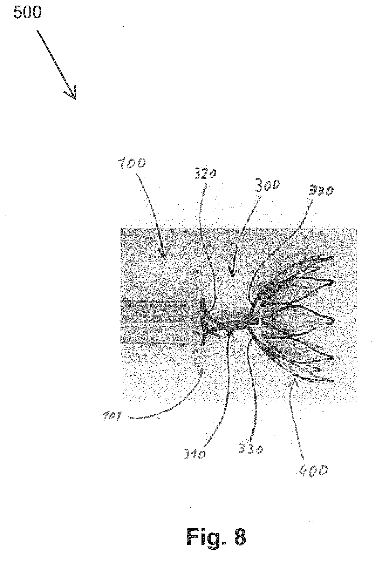

Figure 8 shows a representation of another embodiment of the implant system 500. The duodenal anchor 100 is connected through the connector 300 to the gastric anchor 400 which is formed by a stent. Other parts of the system substantially correspond to the embodiment shown in Fig. 7 and identical parts are not described again for clarity.

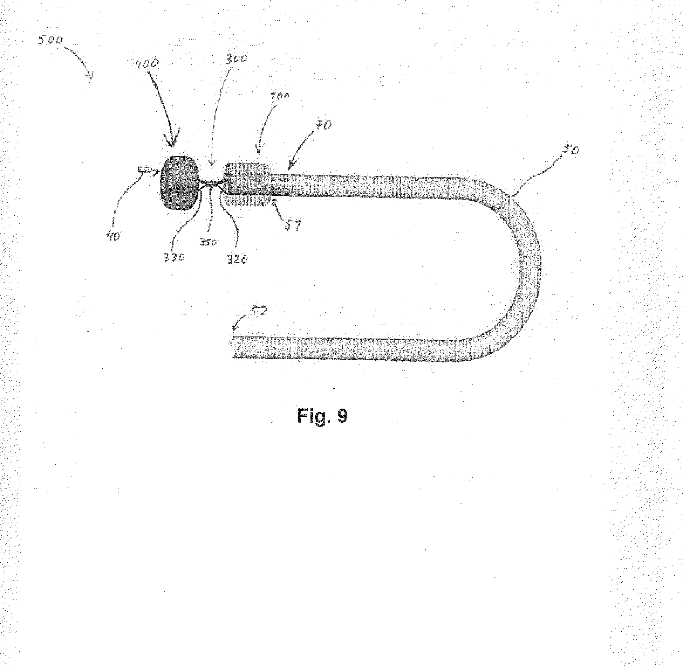

Figure 9 shows a schematic representation of another embodiment of the implant system 500. The gastric anchor 400 is formed as a balloon and connected through the connector 300 to the duodenal anchor 100 comprising elongated elements 70. The elongated elements are arranged inside a sleeve 50. The gastric balloon 400 comprises a valve mechanism 40 for inflation. The duodenal anchor is inflatable by a separate valve mechanism (not shown, see Figs. 2 and 3). Additionally or alternatively, the valve mechanism 40 may also be connected to the inflatable part of the duodenal anchor for inflating said inflatable part. The sleeve 50 has a distal end 52 and a proximal end 51. The proximal end 51 of the sleeve 50 is connected to the duodenal anchor 100 such that the elongated elements 70 are inside the sleeve 50 at the proximal end 51 of the sleeve 50.

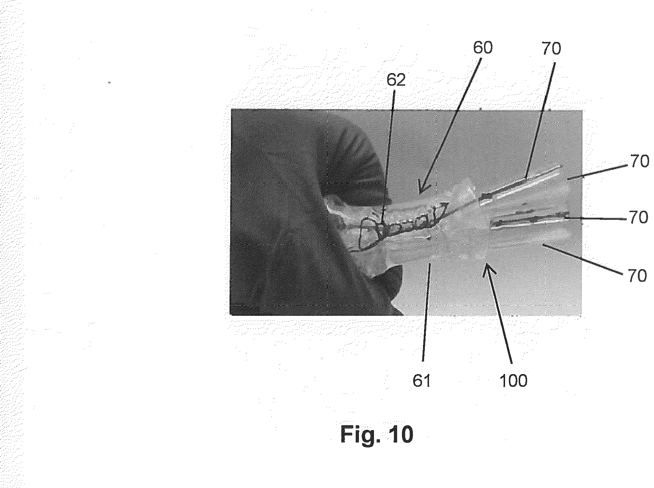

Figure 10 shows a duodenal anchor 100 according to the invention in a crimped state. The duodenal anchor 100 is crimped by a release mechanism 60 comprising a silicone crimping membrane 61 and a release wire 62. The silicone crimping membrane 61 of the release mechanism 60 is placed around the duodenal anchor 100, is pulled together such that it applies a concentric force to the duodenal anchor 100 and kept in this configuration by the release wire 62. Release wire can be selectively released, independent of inflation, by an operator such as to expand the duodenal anchor 100.

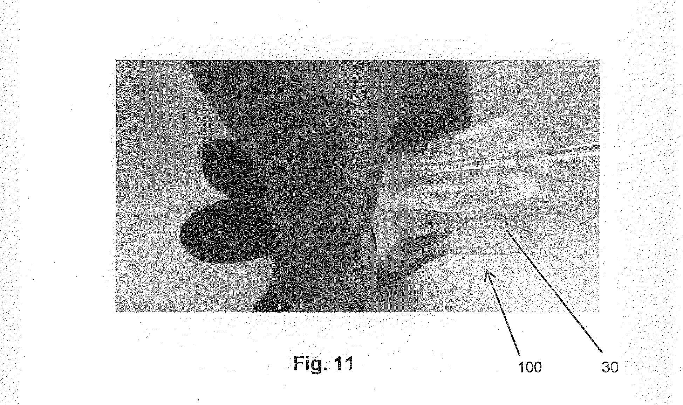

Figure 11 shows the duodenal anchor 100 of Fig. 10 after release of the release wire and removal of the silicone jacket (both not shown, see Fig. 10). The inflatable part 30 of the duodenal anchor 100 is in a deflated state, i.e. there is no or very few liquid and/or gas inside the inflatable part 30 of the duodenal anchor 100.

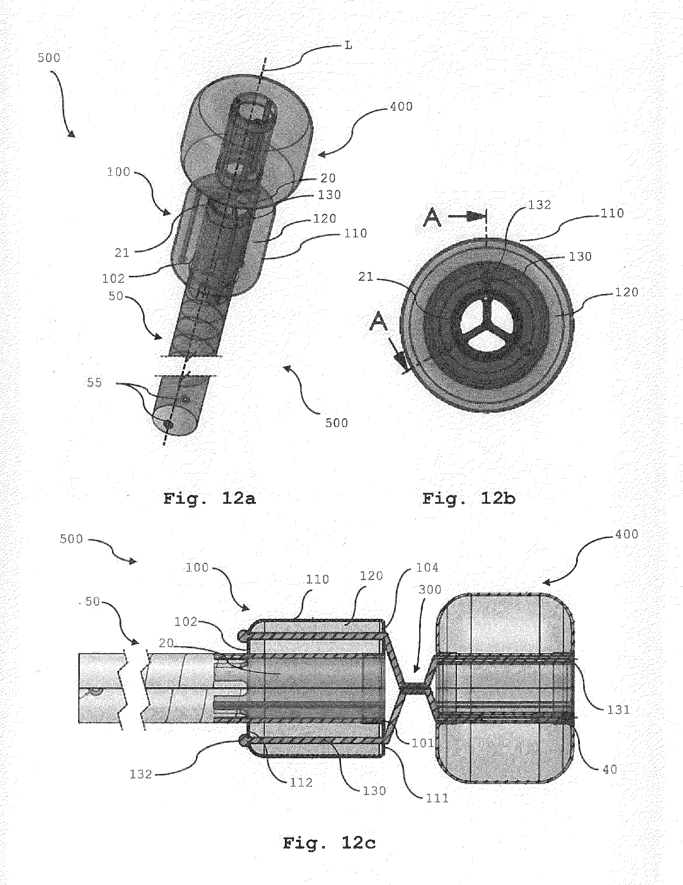

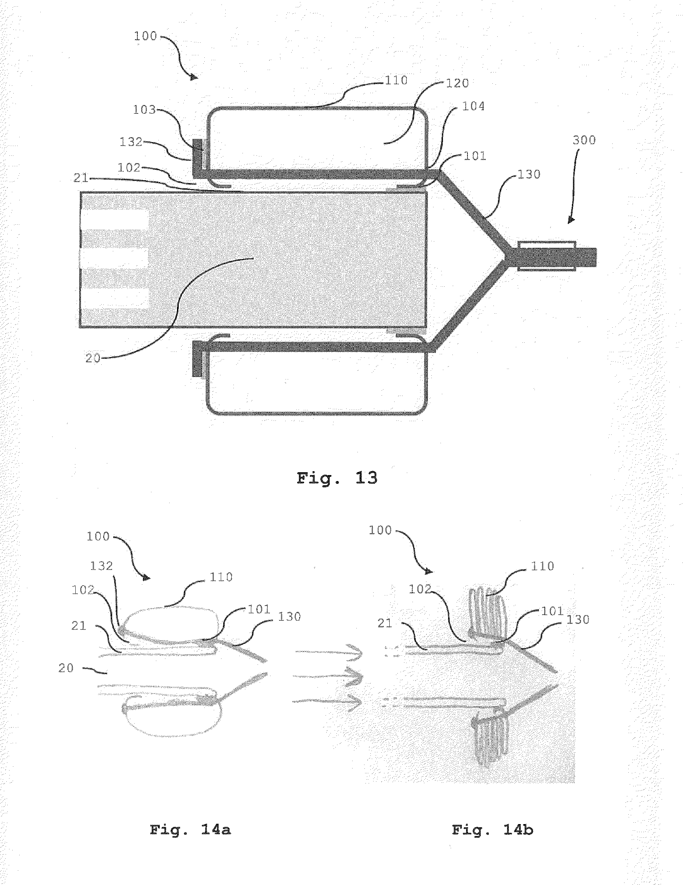

Figure 12a shows an implant system 500 having a gastric anchor 400, a duodenal anchor 100, and a sleeve 50. A connector (see Fig. 12c) connects the gastric anchor and the duodenal anchor 100. The sleeve 50 and its connection to the duodenal anchor 100 are substantially as shown in Figs. 7 and 9 and are not described again for clarity. Here, the duodenal anchor 100 is formed by an inner passage 20 having a wall 21 and an outer jacket 110 arranged circumferentially around inner passage 20. The arrangement of the outer jacket 110 with respect to the inner passage 20 will be described in more detail in Figs. 12c and 13. As shown here, extensions 130 of connector extend through an inner volume 120 formed between the outer jacket 110 and the inner wall 21. When implanted, substantially in the shown configuration, the gastric anchor 400 is positioned in the patient's stomach and allows chyme to pass through. The duodenal anchor 100 is positioned in the patients duodenum and may receive chyme which passed through the gastric anchor 400 and the patient's pylorus and then exits the implant assembly 500 via sleeve 50. The chyme generally passes through the implant assembly 500 along the longitudinal axis L. Here, the outer jacket 110 is not connected to the wall 21 at a distal end and thus forms a gap 102 which allows the jacket 110 to slide along the outer wall 21. Two radio-opaque markers 55 are arranged at a distal end of sleeve 50 to facilitate placement and/or check-ups on the positioning of the implant.

Fig. 12b shows a perspective view of the duodenal anchor 100 in a direction parallel to the longitudinal axis L (see Fig. 12a) and from distal to proximal (i.e. in the opposite direction of chyme transport, when implanted and used as intended). Distal ends 132 of connector extensions 130 are placed on an outside surface of jacket 110 and provide attachment due to a diameter which is larger than a hole through which connector extensions 130 pass. Extensions 130 are elastic wires made out of silicone.

Fig. 12c shows a cross-sectional view of the implant assembly 500 of Fig. 12a along plane A of Fig. 12b. The gastric anchor 400 is an inflatable anchor which may be filled with a gas and/or a fluid via valve mechanism 40 which is embedded in the shaft of the gastric anchor 400. Extensions 131 of connector 300 extend through the gastric anchor 400 in an inner wall of gastric anchor. Connector 300 is substantially identical as shown in e.g. Figs. 7 and 8 and is formed by extensions 131, 130, which extend through gastric anchor 400 and duodenal anchor 100, and are joined to a single strand in a middle portion. Sleeve 50, which is attached to the duodenal anchor 100, substantially corresponds to e.g. the configuration of Fig. 9. To this end, the duodenal anchor 100 comprises a so-called castle-like structure having elongations 70 and gaps 71 in between formed in the wall 21 of inner tube 20. The duodenal anchor further comprises an outer jacket 110 which is glued to the wall 21 at a proximal anchor point 101, generally arranged on a proximal side 111 of duodenal anchor 100, and forms a fluid-tight seal. The outer jacket 110 has a thickness of 0.6 mm. Extension 130 of connector passes through outer jacket 110, through a passage hole, in proximity of anchor point 101 and generally on a proximal side of the duodenal anchor. The extension 130 is slidably arranged in the passage hole 104 and is substantially fluid-tight here, but it will be understood that fluid passage through the passage may be acceptable. At a distal side 112 of the duodenal anchor 100, extension 130 passes through a second, distal passage hole in outer jacket 110 and held by distal bulb 132 of extension 130. Additionally or alternatively, an adhesive may be employed to secure the extension.130 to the outer jacket 110. The outer jacket 110 is not secured to the wall 21 at a distal side 112, thus forming gap 102 which extends around circumference of wall 21 and allows sliding of outer jacket 110 along the wall 21. As a result, and as will be shown in Fig. 14b in more detail, the outer jacket 110 may move in a proximal direction and reduce inner volume 120.

Fig. 13 shows, in a schematic way, the configuration of outer jacket 110 and inner tube 20 of the duodenal anchor 100 of Fig. 12a-12c. Connector 300 and inner tube 20 correspond to previous embodiments and will not be described again for clarity. Here, the outer jacket 110, which forms inner volume 120 with wall 21 of inner tube 20, is attached to the wall 21 at a proximal location via a first adhesive layer 101. It will be understood that adhesive layer 101, which is visible as a cross-section here, extends around the full circumference of wall 21 and forms a fluid-tight seal at the proximal end of the duodenal anchor 100. Extension 130 of anchor 300 extends through the inner volume 120. It will be understood that here, two extensions 130 are visible in accordance with plane A of Fig. 12b, but three extensions 130 are present in this embodiment and are spaced at equal angles around the longitudinal axis of the duodenal anchor 100. The extension 130 passes through a hole 104 of outer jacket 110 at a proximal side and is slidably arranged therein. At a distal side of the duodenal anchor 100, the extension 130 passes through outer jacket 110, where a bulb 132 and is fixed to an outside surface of the outer jacket 110 via a second adhesive layer 103. At a distal end of the outer jacket 110, and generally in the region of the attachment of extension 130 to outer jacket 110, the outer jacket 110 is not attached to the wall 21 of inner tube 20 and forms a gap 102. Due to the gap 102, which extends around the circumference of inner tube 20, the outer jacket 110 is slidable in a proximal direction when a pulling force is exerted on extension. The proximal end of outer jacket 110, due to attachment 101, is fixed to inner tube 20 and does not move with respect to wall 21 when extension 130 is pulled back and distal end of jacket 110 is moved in a proximal direction.

Fig. 14a shows, schematically, a duodenal anchor 100 similar to the duodenal anchor of Fig. 13 in an expanded configuration. Here, the extension 130 is only attached to the outer jacket 110 via bulb 132 but without additional adhesive (see Fig. 13) on the distal side. A proximal end is attached to wall 21 via an adhesive layer 101. The gap 102 allows sliding of the outer jacket 110 on wall 21 of tube 20. Extension 130 extends through opening 104 on the proximal side of outer jacket 110.

Fig. 14b shows the duodenal anchor 100 of Fig. 14b when the extension 130 has been pulled back. As explained above, the gap 102 allows sliding to that the distal end of outer jacket 110 is moved along wall 21 while the adhesive layer 101 fixes proximal end on wall 21. The outer jacket 110 is therefore pulled back and forms a folded, accordion-like structure as the extension 130 is pulled back through opening 104 in outer jacket 110. Filling liquid (not shown) may escape via gap 102. Due to the accordion-like structure, an increased anchoring in the duodenum may be achieved.

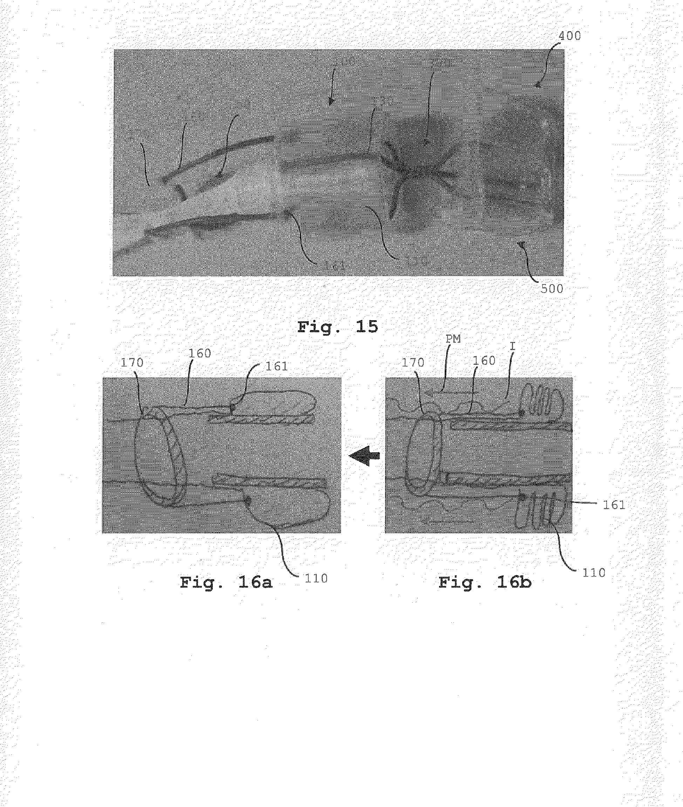

Fig. 15 shows an embodiment of an implant system 500 similar to previously shown embodiments. The gastric anchor 400, duodenal anchor, connector 300 and sleeve 50 are substantially identical to the embodiment of Fig. 12c, but it will be understood that any of these elements may be identical to any of the previously described configurations. In particular, the duodenal anchor 100 need not have a gap and thus correspond to e.g. the embodiment of Fig. 9. Here, a ring 170 is attached to outer jacket 110 via three rods 160. The rods 160 may be made from the same material and same thickness as the extensions 130 of connector 300. Rods are attached to the outer jacket 110 via holes with bulbs 161 arranged on an inside surface of outer jacket 110 in between the ends of extensions 130 attached thereto. It will be understood that the rods 160 may be attached by any means known in the art and at any-location generally on a distal side of outer jacket 110. It is also conceivable to configure extensions 130 to extend beyond the end of outer jacket 110 so as to form, integrally, rods 160 and connect to ring 170.

Figs. 16a and 16b schematically illustrate the function of ring 170.

Fig. 16a shows the implant system 500 of Fig. 15, when implanted, in an expanded configuration corresponding to the configuration of Fig. 14a.

Fig. 16b shows the implant system 500 of Fig. 15, when implanted, in a folded configuration corresponding to the configuration of Fig. 14b. Intestine I performs natural peristaltic movements, symbolically shown by arrow PM, which interact with ring 170 and exert pulling force on ring 170. Ring 170 is generally larger than sleeve 50 so as to surround the sleeve 50. As a result, the ring 170 pulls back the outer jacket 110, via rods 160 with bulb 161. The ring 170 and rods 160 therefore act as a pulling device. While a pulling force acting on the duodenal anchor may generally be desired in certain circumstances, it is particularly advantageous to provide this type of pulling device when the outer jacket 110 is not attached a distal end and thus folds up when a pulling force is exerted on the extensions 130, as described above. The pulling device may then act as a counterforce which re-opens and/or dampens the folding of outer jacket 110 and thus provides better adaptation of the duodenal anchor 100 to the patients' intestine. Rods 160 are formed by inelastic wire cores surrounded by medical-grade silicone. Therefore, the rods 160 are atraumatic and provide biocompatibility while being adapted for transmitting the pulling force. Furthermore, the increased rigidity provided by inelastic core wires may prevent bending, turning and/or flipping over of ring 170. Rods 160 have a length adapted such that ring 170 is positioned distally of duodenal anchor 100 even when jacket 110 is fully folded up.

## Claims

1. A duodenal anchor (100) for holding in place an implant in a duodenum of a patient, having an outer surface (10, 31) which is configured to adapt to an inner wall of the patient's duodenum, the duodenal anchor (100) comprising:
- an inner, preferably central, passage (20) for conveying chyme, and
- a fillable part (30) arranged at least partially circumferentially with respect to the inner passage (20), **characterized in that** the fillable part (30), when fully filled with a fluid, has a larger circumference than an inner circumference of the duodenum.

2. The duodenal anchor (100) according to claim 1, wherein the fillable part is formed by an outer jacket (110) having a first end (111) and a second end (112), wherein the first end (111) is attached, preferably glued, to the inner passage (20) such as to form an inner volume (120).

3. The duodenal anchor (100) according to claim 2, wherein the second end (112) is a free end adapted to move at least, preferably only, along a surface of the inner passage (20).

4. The duodenal anchor (100) according to any one of the preceding claims, wherein at least one connector (130), preferably three connectors (130), passes through the fillable part (30) and is attached to a distal end (112) of the fillable part (30), preferably the second end (112) of the outer jacket (110).

5. The duodenal anchor (100) according to any one of the preceding claims, further comprising a pulling device (160, 170) attached to the distal end (112) of outer jacket (110) .

6. The duodenal anchor (100) according to any one of the preceding claims, wherein the duodenal anchor (100) has a cylindrical shape over at least part of its length, preferably over more than 40 % of its length, in a direction substantially parallel to a longitudinal axis (L) of the duodenal anchor (100).

7. The duodenal anchor (100) according to any one of claims 1 to 3, wherein the duodenal anchor (100) comprises a valve mechanism (40, 41) connected or connectable to the fillable part (30) for filling the fillable part (30).

8. The duodenal anchor (100) according to any one of the preceding claims, wherein the duodenal anchor (100) is attached to a proximal end (51) of a sleeve (50), the sleeve having a distal end (52) for placement within the small intestine.

9. The duodenal anchor (100) according to any one of the preceding claims, wherein the inner passage (20) is substantially tube shaped.

10. The duodenal anchor (100) according to any one of the preceding claims, wherein the fillable part (30) comprises a mechanically flexible outer wall (31).

11. The duodenal anchor (100) according to any one of the preceding claims, wherein the duodenal anchor (100), preferably the fillable part (30), is crimped together by a release mechanism (60), preferably a crimping membrane (61) and a release wire (62), particularly preferably a silicone crimping membrane, to reduce a radial size of the duodenal anchor (100) for delivery.

12. The duodenal anchor (100) according to any one of the preceding claims, wherein the duodenal anchor (100) comprises elongated elements (70, 270) with free distal ends (72, 272) that are extending in a direction substantially parallel to the longitudinal axis (L) of the duodenal anchor (100) .

13. The duodenal anchor (100) according to any one of the preceding claims, wherein the duodenal anchor (100) is connectable, at a proximal end (101), to one of a connector (300) and a stent (400), preferably by a plurality of connection elements (82) arranged at a circumferential position with respect to the central passage (20).

14. An implant (200) for placement in a duodenum of a patient, having a generally tube-shaped implant body (210) with a proximal end (211) and a distal end (212), wherein the proximal end (211) is fixable or fixed to a duodenal anchor, preferably a duodenal anchor (100) according to any one of the preceding claims, and wherein the implant body (210) comprises a plurality of elongated elements (220) with free distal ends (272) extending in a direction substantially parallel to a longitudinal axis (L) of the implant body (210), such that the free ends (72, 272) of the elongated elements (70, 270) form a distal-most portion of the implant body (210).

15. The implant (200) according to claim 14, wherein the implant (200) further comprises a sleeve (50) arranged around the tube-shaped implant body (210) and attached to the proximal end (211) of the implant body (210), wherein a distal end (52) of the sleeve (50) is configured for placement within the small intestine.

16. The implant (200) according to claim 14 or 15, wherein the implant body (210) comprises 3 to 20, preferably 6 to 12, elongated elements (70, 270).

17. The implant (200) according to claims 14 to 16, wherein the implant (200) comprises or consists of a soft material, preferably an elastic soft material.

18. The implant (200) according to claims 14 to 17, wherein the implant (200) has an outer dimension (D), in a direction perpendicular to a longitudinal axis (L), between 1 and 10 cm.

19. The implant (200) according to claims 14 to 18, wherein the elongated elements (70, 270) have a closed surface (73), in particular in a direction perpendicular to a longitudinal axis (L)

20. The implant (200) according to claims 14 to 19, wherein at least one circumferential space (271) between the elongated elements (70, 270) has a width corresponding to the width of the elongated element (70, 270).

21. The implant (200) according to claims 14 to 20, wherein the elongated elements (70, 270) are impermeable to liquids and gases.

22. The implant (200) according to claims 14 to 21, wherein the generally tube-shaped implant body (210) has walls without openings.

23. A connector (300) for connecting a duodenal anchor (100) and a gastric anchor (400), preferably wherein the duodenal anchor (100) is a duodenal anchor (100) according to any one of claims 1 to 13,
having a central portion (310) and a first (320) and second plurality of extremities (330) arranged at opposite ends of the central portion (310), the central portion (310) having a generally elongated shape along a longitudinal axis (L)
wherein the first plurality of extremities (320) is configured to be fixed or fixable to the duodenal anchor (100) at first extremity free ends (321), wherein the second plurality of extremities (330) is configured to be fixed or fixable to the gastric anchor (400) at second extremity free ends (331),
wherein the first plurality (321) and second plurality free ends (331) extend away, in a radial direction, from the longitudinal axis (L) and preferably have connecting means for connection to a duodenal anchor (100) and a gastric anchor (400), respectively.

24. The connector (300) according to claim 23, wherein at least one of the first (320) and second plurality of extremities (330) is formed by rods, preferably exactly three rods.

25. The connector (300) according to claim 23 or 24, wherein at least one of the first plurality free ends (321) and the second plurality free ends (331) are arranged at substantially constant angles and/or radial distances with respect to the longitudinal axis (L).

26. The connector (300) according to claims 23 to 25, wherein the central portion (310), in a direction along the longitudinal axis (L), extends over a length between 3 and 50 mm, preferably between 20 and 30 mm.

27. The connector (300) according to claims 23 to 26, wherein the lengths of the first plurality of extremities (320) and/or the second plurality of extremities (330) is between 10 and 50 mm, preferably between 20 and 30 mm.

28. The connector (300) according to claim 23 or 27, wherein the first plurality of extremities (320) and/or the second plurality of extremities (330) and/or the central portion (310) comprise or consist of a soft material.

29. The connector (300) according to any one of claims 23 to 28, wherein the central portion (310) is formed by a plurality of central extensions (340) and a tubular element (350) arranged circumferentially around the central extensions, preferably such as to bring the central extensions (340) into physical contact with each other.

30. An implant system (500) for implantation in a patient's gastric tract, comprising
a gastric anchor (400), preferably one of a stent and a balloon, adapted to be placed in a patient's stomach,
a duodenal anchor (100), preferably a duodenal anchor (100) according to any one of claims 1 to 13,
a connector (300), preferably a connector (300) according to any one of the claims 23 to 29, having a first (360) and a second end (370) and a central portion (310),
wherein the connector (300) is attached to the duodenal anchor (100) at a first end (360), and connected to the gastric anchor at the second end (370),
further wherein the first (360) and second ends (370) of the connector (300) are connected by a central portion (310) adapted to be placed, when the system (500) is implanted, across a patient's pylorus to connect the gastric anchor (400) and the duodenal anchor (100).

31. The system (500) according to claim 30, further comprising an implant (200), preferably an implant (200) according to 10 to 18, with an implant body (210), wherein the implant body (210) is attached to the duodenal anchor (100) at a proximal end (101) and preferably wherein the implant body (210) comprises elongated elements (70) extending in a direction away from the duodenal anchor (100).

32. The system (500) according to any one of claims 30 to 31, further comprising a sleeve (50) arranged around the tube-shaped implant body (210) and attached to the proximal end (211) of the implant body, wherein a distal end of the sleeve (50) is configured for placement within the small intestine.

33. A method of treating a patient comprising the implantation of a system (500) according to claims 30 to 32.

34. A method of treating a patient, comprising the steps of placing a duodenal anchor (100) comprising an fillable part (30), preferably a duodenal anchor (100) according to claims 1 to 9, in a patient's duodenum, further comprising a step of filling a predetermined quantity of fluid into the fillable part (30), wherein the predetermined quantity is between 10% and 80% of a volume of the fillable part when fully inflated.

35. The method of treating a patient according to claim 34, wherein the predetermined quantity is between 5 and 10 ml.
